# EUROPEAN PATENT SPECIFICATION

(11) **EP 2 070 926 B1**
(45) Date of publication and mention of the grant of the patent: **07.09.2011**
(21) Application number: 07816496.9
(22) Date of filing: 30.09.2007
(51) Int. Cl.: C07D 455/03, A61K 31/4375, A61P 3/04, A61P 3/06, A61P 3/10

(54) **13,13A-DIHYDROBERBERINE DERIVATIVES, THEIR PHARMACEUTICAL COMPOSITION AND USE**
13,13A-DIHYDROBERBERINDERIVATE, IHRE PHARMAZEUTISCHEN ZUSAMMENSETZUNGEN UND VERWENDUNG
DÉRIVÉS DE 13,13A-DIHYDROBERBÉRINE, LEUR COMPOSITION PHARMACEUTIQUE ET LEURS UTILISATIONS

(30) Priority: 30.09.2006 CN 200610140429
(43) Date of publication of application: 17.06.2009
(73) Proprietor: Shanghai Institute of Materia Medica, Chinese Academy of Sciences, Shanghai 201203 (CN)
(72) Inventor: HU, Lihong, Shanghai 201203 (CN); YE, Jiming, Darlinghurst, New South Wales 2010 (AU); LI, Jingya, Shanghai 201203 (CN); JAMES, David, E, Darlinghurst, New South Wales 2010 (AU); KRAEGEN, Edward, W, Darlinghurst, New South Wales 2010 (AU); ZHANG, Hankun, Shanghai 201203 (CN); CHENG, Zhe, Shanghai 201203 (CN); LI, Jia, Shanghai 201203 (CN)
(74) Representative: HOFFMANN EITLE
(86) International application number: PCT/CN2007/002882
(87) International publication number: WO 2008/040192

(56) References cited:
- WO-A-2006/029577
- CN-A- 1 295 573
- CN-A- 1 330 650
- CN-A- 1 393 264
- CN-A- 1 582 930
- CN-A- 1 629 160
- JP-A- 09 095 452
- US-A1- 2003 220 301
- US-A1- 2005 019 435
- US-B1- 6 239 139
- DATABASE CA [Online] CHEMICAL ABSTRACTS SERVICE, COLUMBUS, OHIO, US; 1983, BODOR, NICHOLAS ET AL: "Improved delivery through biological membranes. XV. Sustained brain delivery of berberine" XP002571399 retrieved from STN Database accession no. 1983:510645 & EUROPEAN JOURNAL OF MEDICINAL CHEMISTRY , 18(3), 235-40 CODEN: EJMCA5; ISSN: 0009-4374, 1983,
- DATABASE CA [Online] CHEMICAL ABSTRACTS SERVICE, COLUMBUS, OHIO, US; 1984, BREWSTER, MARCUS ET AL: "Improved delivery through biological membranes. XVI. Enhancement of the brain-specificity of a dihydropyridine .dblarw. pyridinium salt drug delivery system by controlled infusion" XP002571400 retrieved from STN Database accession no. 1984:91246 & JOURNAL OF PARENTERAL SCIENCE AND TECHNOLOGY , 37(5), 159-64 CODEN: JPATDS; ISSN: 0279-7976, 1983,
- BODOR N ET AL: "Improved delivery through biological membranes. XV. Sustained brain delivery of berberine" CHIMIE THERAPEUTIQUE, EDITIONS DIMEO, ARCUEIL, FR, vol. 18, no. 3, 1 January 1983 (1983-01-01), pages 235-240, XP008111414 ISSN: 0009-4374
- BODOR N ET AL: "Problems of delivery of drugs to the brain" PHARMACOLOGY AND THERAPEUTICS, ELSEVIER, GB, vol. 19, no. 3, 1 January 1982 (1982-01-01), pages 337-386, XP025563529 ISSN: 0163-7258 [retrieved on 1982-01-01]
- WEI J. ET AL.: 'Clinical study on improvement of type II diabetes mellitus complicated with fatty liver treated by berberine' ZHONGXIYI JIEHE GANBIN ZAZHI vol. 14, no. 6, 2004, pages 334 - 336, XP008105192
- NIGEL ET ALL: DIABETES, vol. 57, 1 January 2008 (2008-01-01), pages 1414-1418,

## Description

### FIELD OF THE INVENTION

The present invention generally belongs to the fields of pharmaceutical chemistry and pharmacotherapeutics. More particularly, the present invention relates to a compound of the following formula (1) 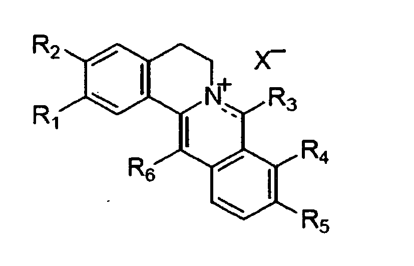 for use in a method for treating diabetes mellitus, adiposity; or fatty liver.

### BACKGROUND OF THE INVENTION

Diabetes mellitus is a group of clinical metabolic diseases induced by the genetic and environmental interactions, and involves a series of disorders of carbohydrate, protein, fat, water and electrolyte metabolisms due to absolute or relative deficiencies in insulin secretion or reduced sensitivity of the target tissues to insulin. Clinically, diabetes mellitus is mainly characterized by hyperglycemia and may lead to damages to multiple organic systems as time goes on, and acute metabolic disorder such as ketoacidosis etc. may occur in serious conditions and stress. The incidence of severe complications such as cardiovascular diseases, kidney damage, diabetic angiopathy and peripheral vascular disease leading to blindness and gangrene etc. in patients suffering from diabetes mellitus is significantly higher than that in persons free of diabetes mellitus. Therefore, diabetes mellitus and complications thereof are the cause of serious public health problems that threaten human health throughout the world.

Diabetes mellitus is mainly classified into two types, namely type 1 diabetes mellitus (insulin-dependent diabetes mellitus, IDDM) and type 2 diabetes mellitus (non-insulin-dependent diabetes mellitus, NIDDM). Presently, over 95% of diabetic patients suffer from type 2 diabetes mellitus. With the improvement of living conditions, the incidence of diabetes mellitus is increasing due to aging, obesity, and participating in unhealthy life style.

The results of a further immunoblotting test showed that AMPK was located in the upstream of p38 MAPK in the cell signaling pathway affected by berberine. The results of the above tests indicated that AMPK was a key target protein by which berberine improved the glucose absorption. Through high performance liquid chromatography, it was found that the AMP:ATP ratio in cells increased significantly, indicating a variation of the energy metabolism levels in cells, which is the key factor of AMPK activation. To sum up, our research showed that berberine improved the glucose absorption by changing the energy metabolism level in cells and thus activating AMPK protein instead of affecting insulin signaling pathway.

In general, berberine has a significant activity of treating diabetes mellitus. However, it has some disadvantageous features such as poor solubility and low oral bioavailability, etc., which restrict the further use of berberine on treating diabetes mellitus. Thus, it is necessary to extensively investigate the relationship between the structure and anti-diabetic activity of berberine, and find out berberine derivatives having better activity and absorption *in vivo.*

### SUMMARY OF THE INVENTION

The present invention is provided to solve the above problems.

An object of the present invention is to provide a compound of the following formula (1) or its physiologically acceptable salt: 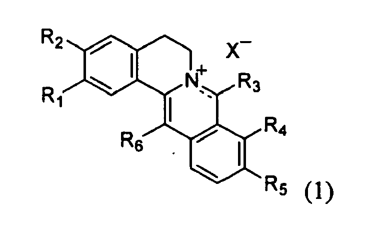 Wherein, "-- " is a double bond or a single bond;
R₁ and R₂ are each independently H, OH, C₁-C₄ alkoxyl or C₁-C₄ acyloxy, or R₁, and R₂ are combined into -O-CH₂-O-;
R₃ is H, C₁-C₄ alkyl, or C₁-C₄ haloalkyl;
R₄ and R₅ are each independently H, OH, C₁-C₄ alkoxyl or C₁-C₄ acyloxy, or R₄ and R₅ are combined into -O-CH₂-O-;
R₆ is H, OH, C₁-C₄ alkyl, C₁-C₄ haloalkyl, an aryl, C₁-C₄ alkoxyl or C₁-C₄ acyloxy,
for use in a method for treating diabetes mellitus, adiposity or fatty liver.

In a preferred embodiment, the present invention is directed to the compounds 19 to 20, 25, and 27 to 41 shown in the following Table III for use in a method for treating diabetes mellitus, adiposity or fatty liver.

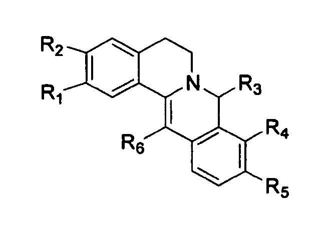

**Table III**

| compound | R₁ | R₂ | R₃ | R₄ | R₅ | R₆ |
|---|---|---|---|---|---|---|
| 19 | -O-CH₂-O- | | H | OCH₃ | OCH₃ | H |
| 20 | -O-CH₂-O- | | CH₃CH₂ | OCH₃ | OCH₃ | H |
| 25 | -O-CH₂-O- | | CCl₃ | OCH₃ | OCH₃ | H |
| 27 | -O-CH₂-O- | | H | OCH₃ | OCH₃ | CH₃ |
| 28 | -O-CH₂-O- | | H | OCH₃ | OCH₃ | CH₃CH₂ |
| 29 | -O-CH₂-O- | | CH₃ | OCH₃ | OCH₃ | H |
| 30 | -O-CH₂-O- | | CH₃ | OCH₃ | OCH₃ | CH₃ |
| 31 | -O-CH₂-O- | | CH₃CH₂ | OCH₃ | OCH₃ | CH₃CH₂ |
| 32 | OH | OH | H | OCH₃ | OCH₃ | H |
| 33 | OH | OH | H | OH | OCH₃ | H |
| 34 | OH | OH | CH₃ | OCH₃ | OCH₃ | H |
| 35 | OH | OH | H | OCH₃ | OCH₃ | CH₃ |
| 36 | OH | OH | CH₃ | OCH₃ | OCH₃⁻ | CH₃ |
| 37 | OAc | OAc | H | OCH₃ | OCH₃ | H |
| 38 | OAc | OAc | H | OAc | OAc | H |
| 39 | OCH₃ | OCH₃ | H | OCH₃ | OCH₃ | H |
| 40 | OCH₃ | OCH₃ | H | OCH₃ | OCH₃ | CH₂CH₃ |
| 41 | -O-CH₂-O- | | H | -O-CH₂-O- | | H |

The compound of formula (1) or its physiologically acceptable salts can be synthesized according to the following process:

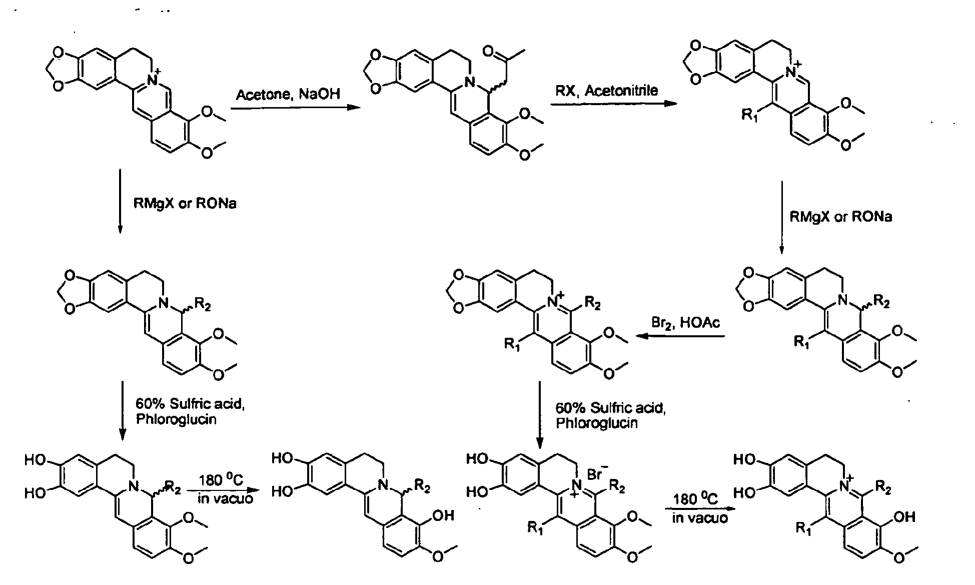

Berberine hydrochloride is dissolved in acetone and treated with a base such as NaOH to obtain acetonylberberine, wherein the 8-site of berberine is attacked by the α-site of acetone. Then, acetonylberberine reacts with a halohydrocarbon under heating to obtain 13-alkylberberine. 13-alkylberberine then reacts with a Grignard reagent to produce a 8,13-dialkyldihydroberberine derivatives. 13-alkylberberine can also react with sodium alcoholate anion under a strong base condition to form 8-alkoxyl-13-alkyldihydroberberine derivatives.

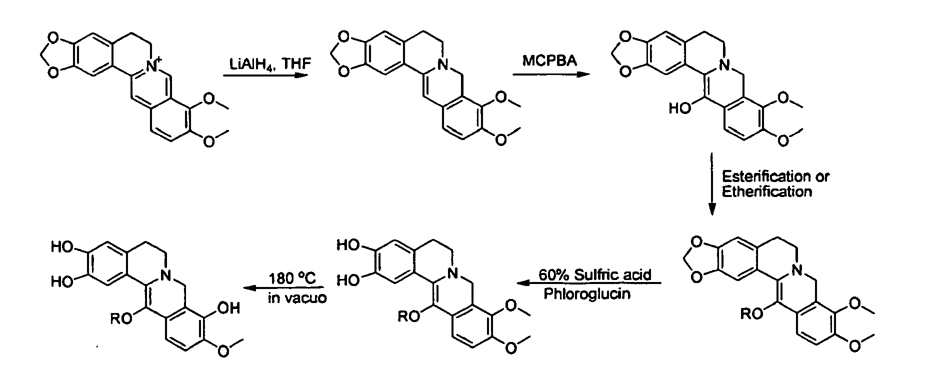

Berberine is reduced into a dihydroberberine with lithium-aluminum hydride in anhydrous tetrahydrofuran. The dihydroberberine is then oxidized with m-chloroperoxybenzoic acid (MCPBA) to obtain a 13-hydroxydihydroberberine. After oxidation, the 13-hydroxydihydroberberine is etherified or esterified to produce a series of 13-alkoxyl or 13-alkanoyloxy dihydroberberines.

Another object of the present invention is to provide a pharmaceutical composition containing a therapeutically effective amount of the compound of the formula (1) depicted above or its physiologically acceptable salt.

### Advantageous Effects:

The present invention designs and synthesizes a compound of the above formula (1), which may promote glucose absorption in muscle cells. The animal tests showed that this series of compounds had effects on improving glucose tolerance and insulin resistance, facilitating weight loss and relieving fatty liver etc. Thus, these compounds can be used to treat diabetes mellitus, adiposity, fatty liver and their complications caused by insulin resistance. The compounds of present invention are easy to prepare and synthesize, and the materials thereof are abundant.

### BRIEF DESCRIPTION OF THE DRAWINGS

Fig. 1 is a graph illustrating the promotion effect of the compound according to the present invention at 5 µM on glucose transport in L6 muscle cells.

Fig. 2A is a graph showing the intraperitoneal glucose tolerance test (ipGTT) after treating the obese mice with compound **19** and its sulfate salt for 2 weeks.

Fig. 2B is a graph showing the incremental areas under the intraperitoneal glucose tolerance test (ipGTT) curves after treating the obese mice with compound **19** and its sulfate salt for 2 weeks.

Fig. 3A is a graph showing the changes in the body weight of mice after the treatment with compound **19** and its sulfate salt for 2 weeks.

Fig. 3B is a graph showing the changes in visceral fat/body weight ratio of mice after the treatment with compound **19** and its sulfate salt for 2 weeks.

Fig. 4A is a graph showing the changes in the content of free fatty acid in blood plasma of mice after the chronic treatment with compound **19** and its sulfate salt for 2 weeks.

Fig. 4B is a graph showing the changes in the content of triglyceride in blood plasma of mice after the chronic treatment with compound **19** and its sulfate salt for 2 weeks.

### DETAILED DESCRIPTION

### Best Mode for Carrying Out the Invention

Hereinafter, the present invention will be described in more detail through the following examples. However, the present invention is not limited to or by the examples.

In the following preparation examples, ¹H-NMR measurement was carried out on a Varian Mercury AMX300 instrument, and MS determination was performed using a VG ZAB-HS or VG-7070 tape as well as Esquire 3000 plus-01005 instruments. All solvents were redistilled before use, and the used anhydrous solvents were subjected to being dried fusing standard methods. Unless otherwise specified, all reactions were carried out under argon atmosphere and tracked by TLC, and each product was washed with saturated sodium chloride solution and dried over anhydrous magnesium sulfate at post-processing. Unless otherwise stated, all products were purified by column chromatography using silica gels, and the used silica gel is GF₂₅₄ with a particle size of 200-300 mesh, which is commercially available form Qingdao Haiyang Chemical Co. Ltd or Yantai Yuanbo Silica Gel Co.

### Preparation Examples

### Preparation example 1: Preparation of compound 19

Berberine (370 mg, 1.0 mmol) was dissolved in 10 mL of anhydrous tetrahydrofuran followed by adding 190 mg of LiAlH₄ (5.0 mmol). The reaction was performed at room temperature under agitation for 2 hours. After the reaction finished, the reaction mixture was evaporated to remove the solvent under reduced pressure, and 0.2 mL of water, 0.2 mL of 30% sodium hydroxide solution and 0.6 mL of water were sequentially added therein. The reaction solution was then filtered, and the filtrate was extracted with ethyl acetate (10 mL x 3). The obtained organic phase was dried over anhydrous sodium sulfate and evaporated under reduced pressure. The residual was purified through silica gel column chromatography (CHCl₃/CH₃OH =50 : 1, eluting until no compound was observed in the eluent) to obtain compound 19(240 mg, 65%).

Compound 19, C₂₀H₁₉NO₄, MW: 337, a yellow amorphous powder, easily dissolved in chloroform and acetone.

¹H NMR (300 MHz, CDCl₃), δ 7.18 (1H, d, J = 8.7 Hz, H-11), 6.73 (2H, m, H-12 and H-1), 6.56 (1H, s, H-4), 5.95 (1H, s, H-13), 5.94 (2H, s, -OCH₂O-) 4.32 (2H, s, H-8), 3.84 (6H, s, OMe x 2), 3.20 (2H, t, J = 8.1 Hz, H-6), 2.90 (2H, t, J = 8.1 Hz, H-5).

### Preparation example 2: Preparation of compound 20

Magnesium strip (240 mg, 10 mmol) and ethyl bromide (1.08 g, 10 mmol) were dissolved in 15 mL of anhydrous diethyl ether under argon atmosphere. The reactant mixture was refluxed for another 2 hours after the violent reaction quieted down. The reaction solution was cooled down to 0 °C, and berberine (370 mg, 1.0 mmol) was slowly added in batch. The ice bath was removed, and the reaction was carried out at room temperature overnight. Then, the reaction solution was injected into ice water (20 mL), and pH of the mixture was adjusted to 5 with a 2 N hydrochloric acid solution. The water phase was separated from the diethyl ether phase, cooled down, adjusted to have a pH of 11~12 with a concentrated ammonia solution, and then extracted with chloroform (20 mL × 3). The obtained organic phase was dried over anhydrous sodium sulfate, and evaporated under reduced pressure. The obtained oil was recrystallized in diethyl ether to obtain compound 20 (220 mg, 59%).

Compound 20, C₂₂H₂₃NO₄, MW: 365, a yellow powder crystal, easily dissolved in chloroform and acetone.

¹H NMR (300 MHz, CDCl₃): δ 7.12 (1H, s, H-1), 6.80 (2H, m, H-11 and 12), 6.57 (1H, s, H-4), 6.00 (2H, s, -OCH₂O-) 5.89 (1H, s, H-13), 3.85 (6H, s, OMe x 2), 3.32 (2H, m, H-6), 3.06 (1H, t, J = 6.0 Hz, H-8), 2.81 (2H, m, H-5), 1.72 (2H, m, H-1'), 0.94 (3H, t, J = 6.3 Hz, H-2').

### Preparation example 3: Preparation of compound 25

Berberine (370 mg, 1.0 mmol) was dissolved in 10 mL of chloroform (analytical grade) under argon atmosphere, followed by adding 0.5 g of sodium hydride. The reaction was carried out under agitation at room temperature overnight. Then, the reaction mixture was evaporated to remove the solvent under reduced pressure, followed by adding 10 mL of water (which had been alkalified with sodium hydroxide). The mixture was then extracted with ethyl acetate (10 mL x 3), and the obtained organic phase was dried over anhydrous sodium sulfate and evaporated under reduced pressure. The obtained yellow powder was recrystallized in methanol to give a yellow needle like crystal 25 (80 mg, 21 %).

Compound 25, C₂₁H₁₈Cl₃NO₄, MW: 453, a yellow needle like crystal, easily dissolved in chloroform and acetone.

¹H NMR (300 MHz, CDCl₃), δ 7.16 (1H, s, H-1), 6.97 (1H, d, J = 8.7 Hz, H-11), 6.86 (1H, d, J = 8.7 Hz, H-12), 6.61 (1H, s, H-4), 6.10 (1H, s, H-13), 6.00 (2H, s, -OCH₂O-), 5.64 (1H, s, H-8), 3.94 (3H, s, OMe-9 or 10), 3.88 (3H, s, OMe-10 or 9), 3.87 (1H, m, H-6α), 3.70 (1H, m, H-6β), 2.89 (2H, m, H-5).

### Preparation example 4: Preparation of compound 27

Compound 2 (480 mg) was dissolved in 20 mL of anhydrous tetrahydrofuran followed by adding LiAlH₄ (120 mg) slowly. The reaction was carried out at room temperature for 3 hours. Water (0.1 mL), 5N sodium hydroxide solution (0.1 mL) and water (0.9 mL) were dropwise added into the reaction solution sequentially. Then, the reaction mixture was filtered, and the filtrate was evaporated to dryness under reduced pressure. The residue was recrystallized in dichloromethane- methanol mixed solvent to give compound 27 (300 mg, 61%).

Compound 27, C₂₁H₂₁NO₄, MW: 351, a yellow powder crystal, easily dissolved in chloroform and acetone.

¹H NMR (300 MHz, CDCl₃): δ 7.17 (1H, s, H-1), 7.03 (1H, d, J = 8.4 Hz, H-11), 6.84 (1H, d, J = 8.4 Hz, H-12), 6.68 (1H, s, H-4), 5.98 (2H, s, -OCH₂O-), 4.33 (2H, s, H-8), 3.86 (6H, s, -OCH₃), 3.13 (2H, m, H-6), 2.83 (2H, m, H-5), 2.78 (3H, s, -CH₃).

### Preparation example 5: Preparation of compound 28

Compound 3 (490 mg) was dissolved in 20 mL of anhydrous tetrahydrofuran followed by adding LiA1H₄ (120 mg) slowly. The reaction was carried out at room temperature for 3 hours. Water (0.1 mL), 5N sodium hydroxide solution (0.1 mL) and water (0.9 mL) were dropwise added into the reaction solution sequentially. Then, the reaction mixture was filtered, and the filtrate was evaporated to dryness under reduced pressure. The residue was recrystallized in dichloromethane- methanol mixed solvent to give compound 28 (320 mg, 63%).

Compound 28, C₂₂H₂₃NO₄, MW: 365, a yellow powder crystal, easily dissolved in chloroform and acetone.

¹H NMR (300 MHz, CDCl₃): δ 7.19 (1H, s, H-1), 7.09 (1H, d, J = 8.4 Hz, H-11), 6.77 (1H, d, J = 8.4 Hz, H-12), 6.69 (1H, s, H-4), 6.01 (2H, s, -OCH₂O-) 4.23 (2H, s, H-8), 3.78 (6H, s, -OCH₃), 3.13 (2H, m, H-6), 2.83 (2H, m, H-5), 2.78 (2H, m, H-1'), 1.34 (3H, t, J = 7.5Hz, H-2').

### Preparation example 6: Preparation of compound 29

Magnesium strip (240 mg, 10 mmol) and methyl iodide (1.40 g, 10 mmol) were dissolved in 15 mL of anhydrous diethyl ether under argon atmosphere. The reaction mixture was refluxed for another 2 hours after the violent reaction quieted down. The reaction solution was cooled down to 0 °C, and berberine (370 mg, 1.0 mmol) was slowly added in batch. The ice bath was removed, and the reaction was carried out at room temperature overnight. Then, the reaction solution was injected into ice water (20 mL), and pH of the mixture was adjusted to 5 with a 2 N hydrochloric acid solution. The water phase was separated from the diethyl ether phase, cooled down, adjusted to have a pH of 11~12 with a concentrated ammonia solution, and then extracted with chloroform (20 mL × 3). The obtained organic phase was dried over anhydrous sodium sulfate, and evaporated under reduced pressure. The obtained oil was recrystallized in diethyl ether to give compound 29 (245 mg, 61%).

Compound 29, C₂₁H₂₁NO₄, MW: 351, a yellow powder crystal, easily dissolved in chloroform and acetone.

¹H NMR (300 MHz, CDCl₃): δ 7.17 (1H, s, H-1), 6.85 (2H, m, H-11 and 12), 6.62 (1H, s, H-4), 6.05 (2H, s, -OCH₂O-), 5.94 (1H, s, H-13), 3.90 (6H, s, OMe × 2), 3.37 (2H, m, H-6), 3.11 (1H, t, J = 6.0 Hz, H-8), 2.86 (2H, m, H-5), 1.31 (3H, t, J =7.0 Hz, H-2').

### Preparation example 7: Preparation of compound 30

Magnesium strip (240 mg, 10 mmol) and methyl iodide (1.40 g, 10 mmol) were dissolved in 15 mL of anhydrous diethyl ether under argon atmosphere. The reaction mixture was refluxed for another 2 hours after violent reaction quieted down. The reaction solution was cooled down to 0 °C, and compound 2 (480 mg, 1.0 mmol) was slowly added in batch. The ice bath was removed, and the reaction was carried out at room temperature overnight. Then, the reaction solution was injected into ice water (20 mL), and pH of the mixture was adjusted to 5 with a 2 N hydrochloric acid solution. The water phase was separated from the diethyl ether phase, cooled down, adjusted to have a pH of 11~12 with a concentrated ammonia solution, and then extracted with chloroform (20 mL × 3). The obtained organic phase was dried over anhydrous sodium sulfate, and evaporated under reduced pressure. The obtained oil was recrystallized in diethyl ether to give compound 30 (232 mg, 43%).

Compound 30, C₂₂H₂₃NO₄, MW: 365, a yellow powder crystal, easily dissolved in chloroform and acetone.

¹H NMR (300 MHz, CDCl₃): δ 7.09 (1H, s, H-1), 6.81 (2H, m, H-11 and 12), 6.52 (1H, s, H-4), 6.01 (2H, s, -OCH₂O-), 3.73 (6H, s, OMe × 2), 3.21 (2H, m, H-6), 3.08 (1H, m, H-8), 2.86 (2H, m, H-5), 1.83 (3H, s, H-1'), 1.38 (3H, d, J =6.3 Hz, H-2').

### Preparation example 8: Preparation of compound 31

Magnesium strip (240 mg, 10 mmol) and ethyl bromide (1.08 g, 10 mmol) were dissolved in 15 mL of anhydrous diethyl ether under argon atmosphere. The reaction mixture was refluxed for another 2 hours after the violent reaction quieted down. The reaction solution was cooled down to 0 °C, and compound 3 (490 mg, 1.0 mmol) was slowly added in batch. The ice bath was removed, and the reaction was carried out at room temperature overnight. Then, the reaction solution was injected into ice water (20 mL), and pH of the mixture was adjusted to 5 with a 2 N hydrochloric acid solution. The water phase was separated from the diethyl ether phase, cooled down, adjusted to have a pH of 11~12 with a concentrated ammonia solution, and then extracted with chloroform (20 mL × 3). The obtained organic phase was dried over anhydrous sodium sulfate, and evaporated under reduced pressure. The obtained oil was recrystallized in diethyl ether to give compound 31(220 mg, 43%).

Compound 31, C₂₄H₂₇NO₄, MW: 393, a yellow powder crystal, easily dissolved in chloroform and acetone.

¹H NMR (300 MHz, CDCl₃): δ 7.07 (1H, s, H-1), 6.75 (2H, m, H-11 and 12), 6.51 (1H, s, H-4), 5.98 (2H, s, -OCH₂O-) 3.85 (6H, s, OMe × 2), 3.32 (2H, m, H-6), 3.06 (1H, t, J = 6.0 Hz, H-8), 2.98 (2H, m, H-3'), 2.81 (2H, m, H-5), 1.72 (2H, m, H-1'), 1.33 (3H, t, J = 6.0 Hz, H-4'), 0.94 (3H, t, J = 6.3 Hz, H-2').

### Preparation example 9: Preparation of compound 32

Compound 19 (337 mg, 1.0 mmol) and 1,3,5-trihydroxybenzene (504 mg, 4.0 mmol) were dissolved in 10 mL of 60% sulphuric acid solution (volume ratio). The obtained solution was then heated to 80 °C, and reacted under refluxing for 1 hour. The reaction mixture was cooled down and extracted with ethyl acetate. The obtained organic phase was distilled to dryness using a rotary evaporator, and the residue was purified through a silica gel column (CHCl₃:MeOH= 10:1) to obtain compound 32 (108 mg, 33%).

Compound 32, C₁₉H₁₉NO₄, MW: 325, a brown powder, easily dissolved in chloroform and methanol.

¹H NMR (300 MHz, CDCl₃): δ 6.87 (1H, s, H-1), 6.52 (1H, s, H-4), 6.75 (1H, d, J = 8.1 Hz, H-11), 6.47 (1H, d, J = 8.1 Hz, H-12), 5.88 (1H, s, H-13), 4.27 (2H, s, H-8), 3.76 (6H, s, -OCH₃), 3.01 (2H, t, J = 6.3 Hz, H-6), 2.54 (2H, t, J = 6.3 Hz, H-5).

### Preparation example 10: Preparation of compound 33

Compound 14 (321 mg, 1.0 mmol) and 1,3,5-trihydroxybenzene (504 mg, 4.0 mmol) were dissolved in 10 mL of 60% sulphuric acid solution (volume ratio). The obtained mixture was heated to 80 °C, and reacted under refluxing for 1 hour. The reaction mixture was cooled down and extracted with ethyl acetate. The obtained organic phase was distilled to dryness using a rotary evaporator, and the residue was purified through a silica gel column (CHCl₃:MeOH= 10:1) to obtain compound 33 (98 mg, 31%).

Compound 33, C₁₈H₁₇NO₄, MW: 311, a brown powder, easily dissolved in chloroform and methanol.

¹H NMR (300 MHz, CDCl₃): δ 6.92 (1H, s, H-1), 6.57 (1H, s, H-4), 6.70 (1H, d, J = 8.0 Hz, H-11), 6.41 (1H, d, J = 8.0 Hz, H-12), 5.83 (1H, s, H-13), 4.33 (2H, s, H-8), 3.87 (3H, s, -OCH₃), 3.11 (2H, t, J = 6.3 Hz, H-6), 2.33 (2H, t, J = 6.3 Hz, H-5).

### Preparation example 11: Preparation of compound 34

Compound 29 (351 mg, 1.0 mmol) and 1,3,5-trihydroxybenzene (504 mg, 4.0 mmol) were dissolved in 10 mL of 60% sulphuric acid solution (volume ratio). The obtained mixture was heated to 80 °C, and reacted under refluxing for 1 hour. The reaction mixture was then cooled down and extracted with ethyl acetate. The obtained organic phase was distilled to dryness using a rotary evaporator, and the residue was purified through a silica gel column (CHCl₃:MeOH= 10:1) to obtain compound 34 (103 mg, 32%).

Compound 34, C₂₀H₂₁NO₄, MW: 339, a brown powder, easily dissolved in chloroform and methanol.

¹H NMR (300 MHz, CDCl₃): δ 6.78 (1H, s, H-1), 6.47 (1H, s, H-4), 6.31 (1H, d, J = 8.0 Hz, H-11), 6.23 (1H, d, J = 8.0 Hz, H-12), 4.37 (2H, s, H-8), 3.73 (6H, s, -OCH₃), 3.11 (2H, t, J = 6.3 Hz, H-6), 2.43 (2H, t, J = 6.3 Hz, H-5), 1.79 (3H, s, -CH₃).

### Preparation example 12: Preparation of compound 35

Compound 27 (351 mg, 1.0 mmol) and 1,3,5-trihydroxybenzene (504 mg, 4.0 mmol) were dissolved in 10 mL of 60% sulphuric acid solution (volume ratio). The obtained mixture was heated to 80 °C, and reacted under refluxing for 1 hour. The reaction mixture was then cooled down and extracted with ethyl acetate. The obtained organic phase was distilled to dryness using a rotary evaporator, and the residue was purified through a silica gel column (CHCl₃:MeOH = 10:1) to obtain compound 35 (107 mg, 33%).

Compound 35, C₂₀H₂₁NO₄, MW: 339, a brown yellow powder, easily dissolved in chloroform and methanol.

¹H NMR (300 MHz, CDCl₃): δ 6.77 (1H, s, H-1), 6.45 (1H, s, H-4), 6.29 (1H, d, J = 8.0 Hz, H-11), 6.21 (1H, d, J = 8.0 Hz, H-12), 4.51 (2H, s, H-8), 3.71 (6H, s, -OCH₃), 3.13 (2H, t, J = 6.3 Hz, H-6), 2.41 (2H, t, J = 6.3 Hz, H-5), 1.34 (3H, s, -CH₃).

### Preparation example 13: Preparation of compound 36

Compound 30 (365 mg, 1.0 mmol) and 1,3,5-trihydroxybenzene (504 mg, 4.0 mmol) were dissolved in 10 mL of 60% sulphuric acid solution (volume ratio). The obtained mixture was heated to 80 °C, and reacted under refluxing for 1 hour. The reaction mixture was then cooled down and extracted with ethyl acetate. The obtained organic phase was distilled to dryness using a rotary evaporator, and the residue was purified through a silica gel column (CHCl₃:MeOH= 10:1) to obtain compound 36 (123 mg, 37%).

Compound 36, C₂₁H₂₃NO₄, MW: 353, a brown yellow powder, easily dissolved in chloroform and methanol.

¹H NMR (300 MHz, CDCl₃): δ 6.81 (1H, s, H-1), 6.55 (1H, s, H-4), 6.32 (1H, d, J = 8.0 Hz, H-11), 6.22 (1H, d, J = 8.0 Hz, H-12), 4.43 (2H, s, H-8), 3.70 (6H, s, -OCH₃), 3.34 (2H, t, J = 6.3 Hz, H-6), 2.58 (2H, t, J = 6.3 Hz, H-5), 1.79 (3H, s, -CH₃), 1.35 (3H, s, -CH₃)-**Preparation example 14: Preparation of compound 37**

Compound 32 (325 mg) was dissolved in a mixture solution of 1 mL of pyridine and 1 mL of acetic anhydride. The reaction was carried out under agitation at room temperature overnight. The reaction mixture was then evaporated to remove the solvent under reduced pressure. The residue was purified through silica gel column chromatography (CHCl₃/CH₃OH =20 : 1) to obtain compound 37 (132 mg, 37%).

Compound 37, C₂₃H₂₃NO₆, MW: 409, a brown yellow powder, easily dissolved in chloroform and methanol.

¹H NMR (300 MHz, CDCl₃): δ 7.15 (1H, s, H-1), 6.77 (1H, d, J = 8.1 Hz, H-11), 6.52 (1H, s, H-4), 6.47 (1H, d, J = 8.1 Hz, H-12), 5.93 (1H, s, H-13), 4.42 (2H, s, H-8), 3.84 (6H, s, -OCH₃), 3.13 (2H, t, J = 6.3 Hz, H-6), 2.87 (2H, t, J = 6.3 Hz, H-5), 2.08 (6H, s, -OCOCH₃).

### Preparation example 15: Preparation of compound 38

2,3,9,10-tetrahydroxy dihydropalmatine (297 mg) was dissolved in a mixture solution of 1 mL of pyridine and 1 mL of acetic anhydride. The reaction was carried out under agitation at room temperature overnight. The reaction mixture was then neutralized with a saturated sodium bicarbonate solution and extracted with ethyl acetate. The obtained organic phase was evaporated under reduced pressure, and the residue was purified through silica gel column chromatography (CHCl₃/CH₃OH =25 : 1) to obtain compound 38 (108 mg, 31%).

Compound 38, C₂₃H₂₃NO₆, MW: 409, a brown yellow powder, easily dissolved in chloroform and methanol.

¹H NMR (300 MHz, CDCl₃): δ 7.21 (1H, s, H-1), 6.79 (1H, d, J = 8.1 Hz, H-11), 6.55 (1H, s, H-4), 6.47 (1H, d, J = 8.1 Hz, H-12), 5.91 (1H, s, H-13), 4.51 (2H, s, H-8), 3.17 (2H, t, J = 6.3 Hz, H-6), 2.84 (2H, t, J = 6.3 Hz, H-5), 2.08 (6H, s, -OCOCH₃), 2.06 (6H, s, -OCOCH₃).

### Preparation example 16: Preparation of compound 39

- Palmatine hydrochloride (390 mg, 1.0 mmol) was dissolved in 10 mL anhydrous tetrahydrofuran followed by adding 190 mg of LiAlH₄ (5.0 mmol). The reaction was carried out under agitation at room temperature for 2 hours. After the reaction finished, the reaction mixture was evaporated to remove the solvent under reduced pressure, and 0.2 mL of water, 0.2 mL of 30% sodium hydroxide solution and another 0.6 mL of water were sequentially added therein. The reaction solution was then filtered, and the filtrate was extracted with ethyl acetate (10 mL × 3). The obtained organic phase was dried over anhydrous sodium sulfate and evaporated under reduced pressure. The residue was purified through silica gel column chromatography (CHCl₃/CH₃OH =50 : 1, eluting until no compound was observed in the eluent) to obtain compound 39 (253 mg, 67%).

Compound **39**, C₂₁H₂₃NO₄, MW: 353, a yellow amorphous powder, easily dissolved in chloroform and acetone.

¹H NMR (300 MHz, CDCl₃), δ 7.21 (1H, s, H-1), 7.08 (1H, d, J = 8.4 Hz, H-12), 6.91 (1H, d, J = 8.4 Hz, H-11), 6.67 (1H, s, H-4), 5.76 (1H, s, H-13), 4.33 (2H, s, H-8), 3.90 (6H, s, -OMe × 2), 3.86 (3H, s, -OMe), 3.83 (3H, s, -OMe), 3.18 (2H, t, J = 7.5 Hz, H-6), 2.91 (2H, t, J = 7.5 Hz, H-5).

### Preparation example 17: Preparation of compound 40

Palmatine hydrochloride (415 mg, 1.0 mmol) was dissolved in 10 mL anhydrous tetrahydrofuran followed by adding 190 mg of LiAlH₄ (5.0 mmol). The reaction was carried out under agitation at room temperature for 2 hours. After the reaction finished, the reaction mixture was evaporated to remove the solvent under reduced pressure, and 0.2 mL of water, 0.2 mL of 30% sodium hydroxide solution and another 0.6 mL of water were sequentially added therein. The reaction solution was then filtered, and the filtrate was extracted with ethyl acetate (10 mL x 3). The obtained organic phase was dried over anhydrous sodium sulfate and evaporated under reduced pressure. The residue was purified through silica gel column chromatography (CHCl₃/CH₃OH =50 : 1, eluting until no compound was observed in the eluent) to obtain compound 40 (223 mg, 61%).

Compound 40, C₂₃H₂₇NO₄, MW: 381, a yellow amorphous powder, easily dissolved in chloroform and acetone.

¹H NMR (300 MHz, CDCl₃), δ 7.17 (1H, s, H-1), 7.03 (1H, d, J = 8.4 Hz, H-12), 6.84 (1H, d, J = 8.4 Hz, H-11), 6.68 (1H, s, H-4), 4.27 (2H, s, H-8), 3.92 (3H, s, -OMe ), 3.91 (3H, s, -OMe ), 3.88 (3H, s, -OMe), 3.85 (3H, s, -OMe), 3.08 (2H, m, H-6), 2.81 (4H, m, H-5 and 1'), 1.34 (3H, t, J = 7.5 Hz, H-2').

### Preparation example 18: Preparation of compound 41

Coptisine hydrochloride (355 mg, 1.0 mmol) was dissolved in 10 mL anhydrous tetrahydrofuran followed by adding 190 mg of LiAlH₄ (5.0 mmol). The reaction was carried out under agitation at room temperature for 2 hours. After reaction finished, the reaction mixture was evaporated to remove the solvent under reduced pressure, and 0.2 mL of water, 0.2 mL of 30 % sodium hydroxide solution and another 0.6 mL of water were sequentially added therein. The reaction solution was then filtered, and the filtrate was extracted with ethyl acetate (10 mL × 3). The obtained organic phase was dried over anhydrous sodium sulfate and evaporated under reduced pressure. The residue was purified through silica gel column chromatography (CHCl₃/CH₃OH =50 : 1, eluting until no compound was observed in the eluent) to obtain compound 41 (223 mg, 61%).

Compound 41, C₁₉H₁₅NO₄, MW: 321, a yellow amorphous powder, easily dissolved in chloroform and acetone.

¹H NMR (300 MHz, CDCl₃), δ 7.13 (1H, s, H-1), 7.02 (1H, d, J = 8.4 Hz, H-12), 6.83 (1H, d, J = 8.4 Hz, H-11), 6.64 (1H, s, H-4), 5.98 (2H, s, -OCH₂O-), 5.96 (2H, s, -OCH₂O-), 4.23 (2H, s, H-8), 3.11 (2H, t, J = 7.5 Hz, H-6), 2.81 (2H, t, J = 7.5 Hz, H-5).

### Experimental Examples

### Experimental Example 1:

The effects of some compounds according to the present invention on glucose absorption were preliminarily evaluated *in vitro* using the glucose uptake model in L6 muscle cells.

### Experimental steps:

The completely differentiated L6 muscle cells which were cultured in 24-well plate were washed with PBS one time, starved in a high glucose DMEM culture medium containing 0.2% of BSA for 2 hours, and incubated in a high glucose DMEM culture medium containing 5 µM of the compound of formula (1) and 0.2% of BSA for 2.5 hours. They were then washed twice with a HBS solution containing 5 µM of the compound of formula (1), and incubated for another 0.5 hour in a HBS solution containing 5 µM of the compound of formula (1).

³H-labeled 2-deoxyglucose (dissolved in HBS or KRP to form a 1 mM, 5 µCi/mL temporary working solution) was added into a HBS solution to obtain a solution with a final concentration of 100 µM and 0.5 µCi/mL of the isotope. The cells were incubated at 37 °C for 10 min. The cell incubation solution was then removed quickly, and the cells were placed on ice, rinsed with ice-cold PBS three times, and dried in an oven at 42 °C. 200 µL of 0.1% TritonX-100 was added therein, and the mixture was mildly vibrated at 4 °C for 45 min to lyse the cells. 150 µL of lysate was taken, and 1.1 mL of scintillation fluid was added therein to perform scinticounting. 10µL of lysate was diluted by 10 times to measure the protein concentration using Bradford method. Final result is presented with a unit of pmol/min/mg protein.

### Evaluation standard:

The compound to be measured was dissolved in DMSO. When the concentration is 5 µM, if the calculated glucose intake in the test using the compound of the present invention is higher than that in DMSO blank control and there is significant difference in statistics between the data of the two groups, it can be concluded that the compound of the present invention can promote glucose absorption.

### Experimental Result:

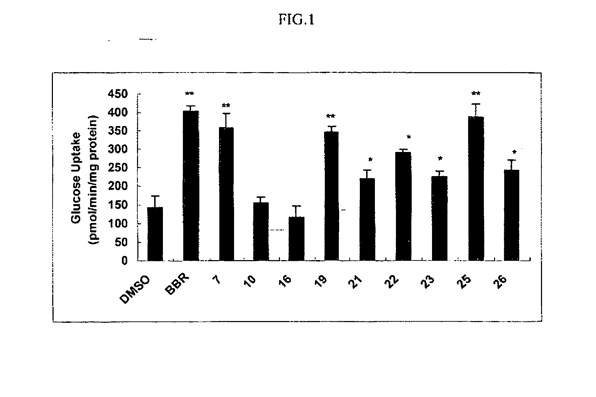

Fig. 1 shows the effects of some compounds of the present invention at 5 µM on promoting glucose transport in 5.0 mM glucose medium in glucose intake model in L6 muscle cells, wherein DMSO is blank control, and BBR is berberine.

It has been found, by screening at cellular level, that several compounds have stronger activity of promoting glucose uptake than berberine. Depending on the difficult level of synthesis, we selected compound **19** and its sulfate salt to perform the pharmacodynamical test at the whole animal level.

### Experiment Example 2:

The *in vivo* antidiabetic activity of the compounds according to the present invention is evaluated.

### Experimental Steps:

Normal male C57BL/6J mice were fed by high-fat diet for 10 weeks to generate obvious symptom of insulin resistance, and their ability of glucose tolerance decreased significantly. Ten mice per group were used to perform the efficacy study of the compounds. Dihydroberberine derivative 19 or its sulfate salt was mixed into the high-fat diet, and administered at a dose of 100 mg/kg/day for 2 weeks. After the mice were starved overnight, basal blood sugar value (0 min) was measured by taking blood from tail vein. Then the mice were injected glucose intraperitoneally at a dose of 2 g/kg according to the body weight of the mice in control group which were fed by normal diet followed by measuring blood sugar value at 15, 30, 45, 60, 90 and 120 min respectively and calculating the area under curve (AUC). The body weight and visceral fat of the mice were weighted, and the contents of fatty acid and triglyceride in the blood plasma of the mice were determined.

### Experimental Result:

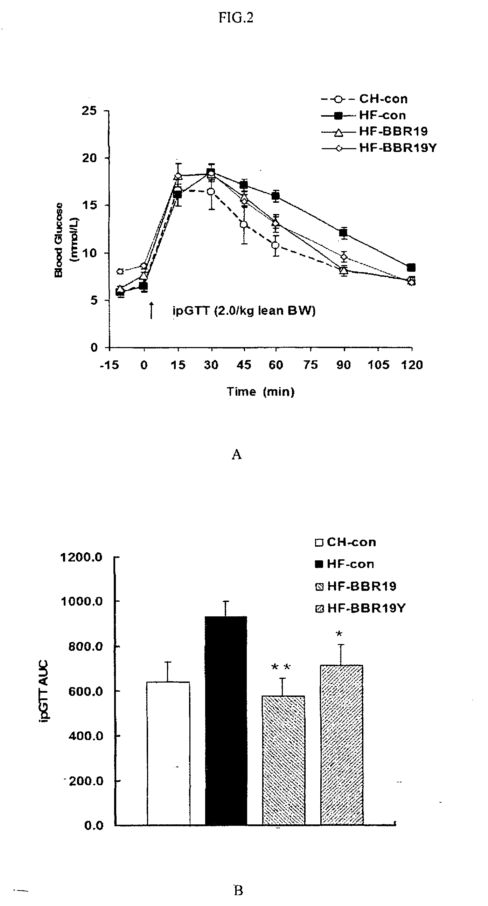

In Figs. 2-4, CH-con means the normal mouse, HF-con refers to the obese mouse, HF-BBR19 is the obese mouse treated with compound 19 for 2 weeks, and HF-BBR19Y is the obese mouse treated with the sulfate salt of compound 19 for 2 weeks.

Fig. 2 shows the intraperitoneal glucose tolerance test (ipGTT) curves of the mice and the areas under curve. The mice which had been treated with compound **19** or its sulfate salt for 2 weeks and the mice in control group were intraperitoneally injected glucose at 2 g/kg after being starved overnight. The blood glucose value was then measured at 0, 15, 30, 45, 60, 90 and 120 min respectively and the area under curve (AUC) was calculated (*, P<0.05; **, P<0.01). It can be seen from Fig. 2 that the obese mice have a significantly improved glucose tolerance after being treated with compound **19** or its sulfate salt for 2 weeks.

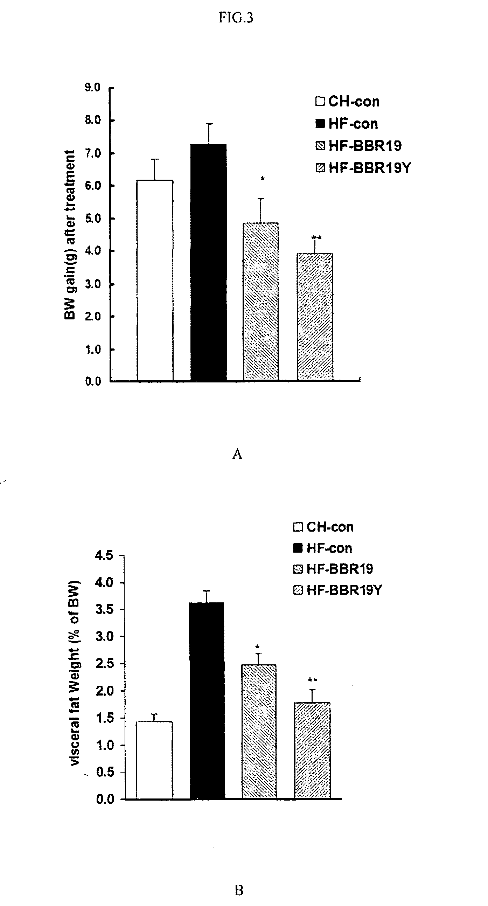

Fig. 3 shows the increase in body weight of the obese mice and their visceral fat/body weight ratio after treatment with compound **19** or its sulfate salt for 2 weeks (*, P<0.05; **, P<0.01). It can be seen from Fig. 3 that the increase in body weight of the mice which had been treated with compound **19** or its sulfate salt for 2 weeks is significantly less than that of the mice in the control group, and the ratio of intra-abdominal fat to body weight is lowered significantly comparing with the control group. Such results indicate that the compounds of the present invention have the effects on preventing the increase in body weight of the mice and fat accumulation induced by high-fat diet. Thus they may have a potential effect on treating obesity.

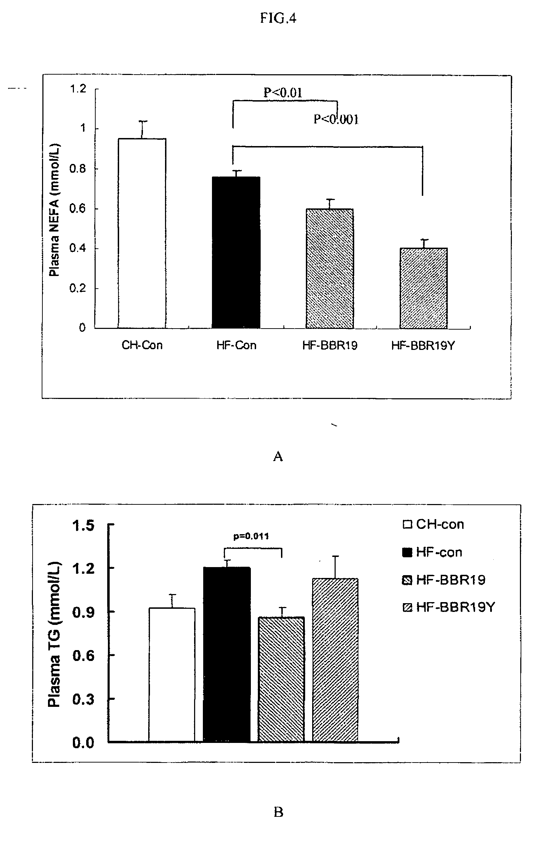

Fig. 4 shows the changes in the contents of free fatty acids (i.e. non-esterified fatty acid, NEFA) and triglyceride (TG) in the blood plasma of obese mice which have been treated with compound **19** or its sulfate salt for 2 weeks (*, P<0.05; **, P<0.01). It can be seen from Fig. 4 that the contents of free fatty acid (NEFA) (HF-Con vs HF-BBR19, 0.76 ± 0.03 mmol/l vs 0.6 ± 0.05 mmol/l and triglyceride TG (HF-Con vs HF-BBR**19**, 1.20 ± 0.05 mmol/l vs 0.86 ± 0.08 mmol/l) in blood plasma of obese mice are both lowered significantly after the mice have been treated with compound **19** or sulfate thereof for 2 weeks.

### Experimental Conclusion:

Compound **19** and its sulfate salt have significant effects on improving glucose tolerance and insulin resistance, facilitating weight loss, lowering the contents of free fatty acids and triglyceride in blood plasma and relieving fatty liver in the mouse models with insulin resistance and obesity induced by a high-fat diet.

## Claims

1. A compound of Formula (1) or its physiologically acceptable salt: 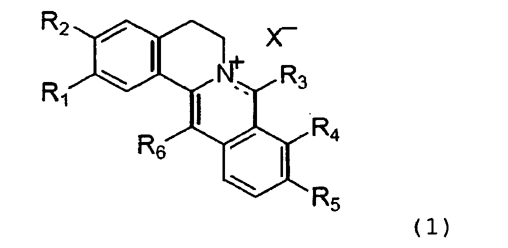 wherein,
"--" is a double bond or single bond;
R₁ and R₂ are each independently H, OH, C₁-C₉ alkoxyl or C₁-C₄ acyloxy, or R₁ and R₂ are combined into -O-CH₂-O-;
R₃ is H, C₁-C₄ alkyl or C₁-C₄ haloalkyl;
R₄ and R₅ are each independently H, OH, C₁-C₄ alkoxyl or C₁-C₄ acyloxy, or R₄ and R₅ are combined into -O-CH₂-O-;
R₆ is H, OH, C₁-C₄ alkoxyl, C₁-C₄ acyloxy, C₁-C₄ alkyl, C₁-C₄ haloalkyl or an aryl,
for use in a method of treating diabetes mellitus, adiposity or fatty liver.

2. The compound of Formula (1) for use in a method according to claim 1, which compound is selected from the following compounds represented by Formulae 19 to 20, 25 and 27 to 41:

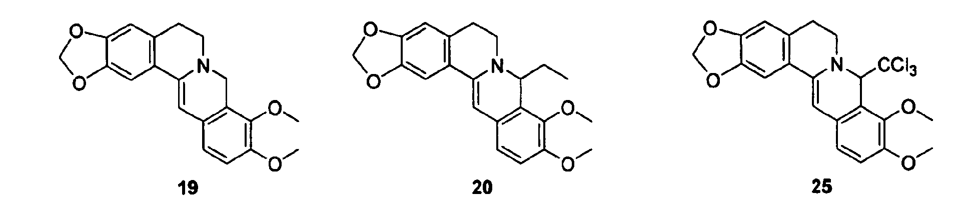

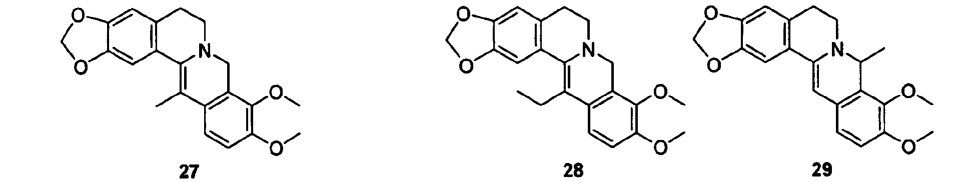

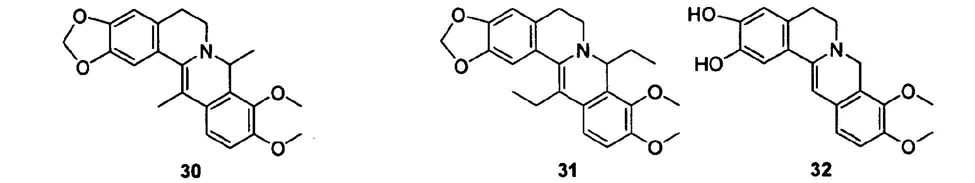

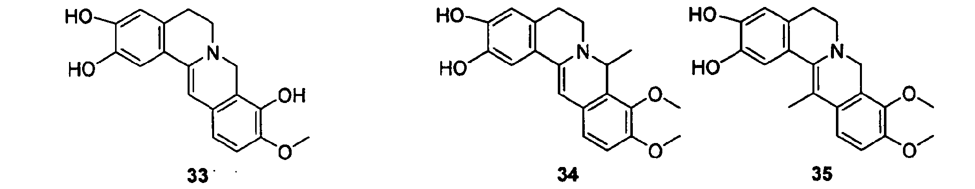

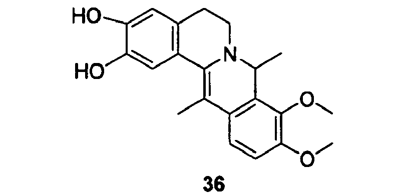

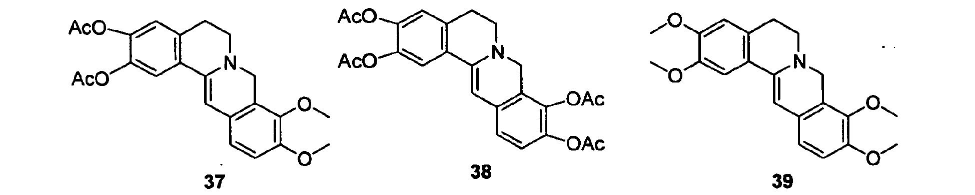

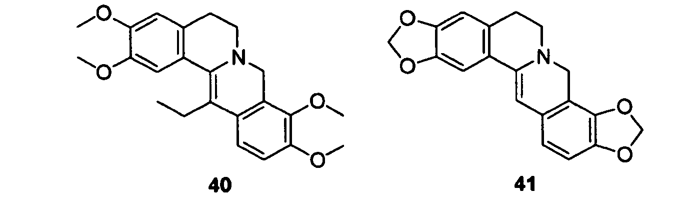

## Patentansprüche

1. Verbindung der Formel (1) oder ihr physiologisch annehmbares Salz: 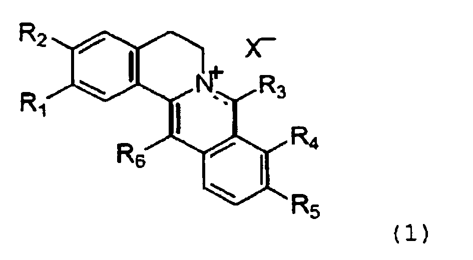 worin:
"--" eine Doppelbindung oder eine Einfachbindung ist,
R₁ und R₂ jeweils unabhängig voneinander H, OH, C₁-₄-Alkoxyl oder C₁₋₄-Acyloxy sind oder R₁ und R₂ in -O-CH₂-O- kombiniert sind;
R₃ H, C₁₋₄-Alkyl oder C₁₋₄-Halogenalkyl ist;
R₄ und R₅ jeweils unabhängig voneinander H, OH, C₁₋₄-Alkoxyl oder C₁₋₄-Acyloxy sind, oder R₄ und R₅ in -O-CH₂-O- kombiniert sind;
R₆ H, OH, C₁₋₄-Alkoxyl, C₁₋₄-Acyloxy, C₁₋₄-Alkyl, C₁₋₄-Halogenalkyl oder Aryl ist,
zur Verwendung in einem Verfahren zur Behandlung von Diabetes mellitus, Fettleibigkeit oder Fettleber.

2. Verbindung der Formel (1) zur Verwendung in einem Verfahren gemäss Anspruch 1, wobei die Verbindung aus den nachstehenden Verbindungen der Formeln 19 bis 20, 25 und 27 bis 41 ausgewählt ist:

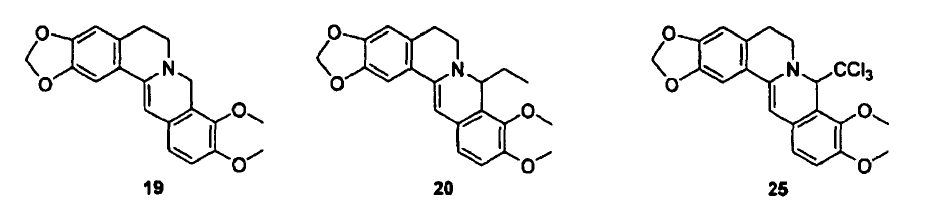

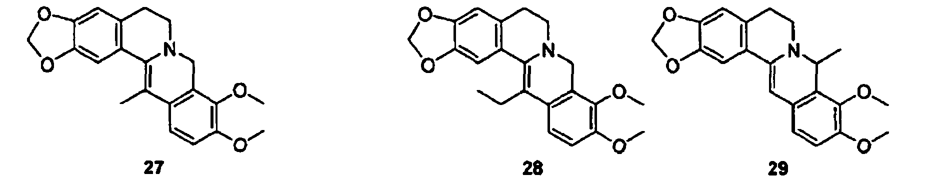

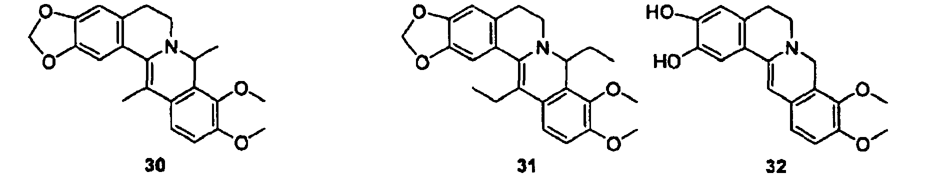

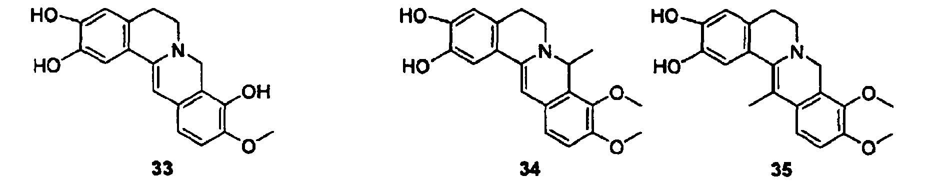

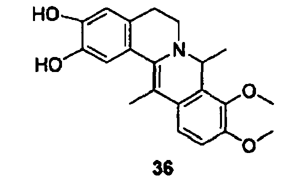

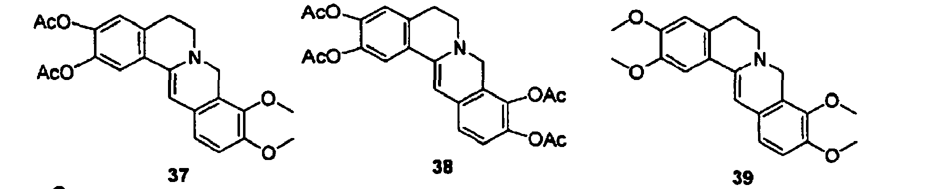

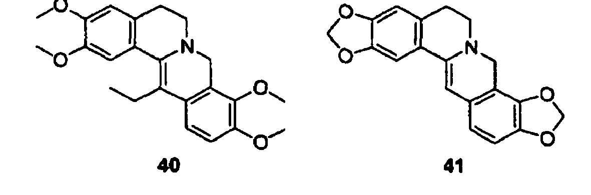

## Revendications

1. Composé de formule (1) ou son sel physiologiquement acceptable : 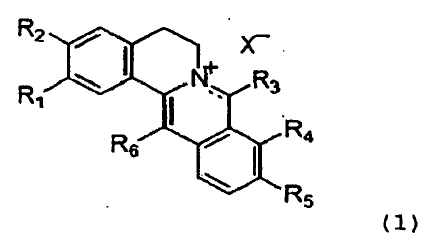 où
"--" est une double liaison ou une simple liaison ;
R₁ et R₂ sont chacun indépendamment H, OH, C₁-C₄ alcoxy ou C₁-C₄ acyloxy, ou R₁ et R₂ sont combinés en -O-CH₂-O- ;
R₃ est H, C₁-C₄ alkyle ou C₁-C₄ haloalkyle ;
R₄ et R₅ sont chacun indépendamment H, OH, C₁-C₄ alcoxy ou C₁-C₄ acyloxy, ou R₄ et R₅ sont combinés en -O-CH₂-O- ;
R₆ est H, OH, C₁-C₄ alcoxy, C₁-C₄ acyloxy, C₁-C₄ alkyle, C₁-C₄ haloalkyle ou un aryle,
destiné à être utilisé dans un procédé de traitement du diabète sucré, de l'adiposité ou de la stéatose hépatique.

2. Composé de formule (1) destiné à être utilisé dans un procédé selon la revendication 1, lequel composé est choisi parmi les composés suivants représentés par les formules 19 à 20, 25 et 27 à 41 :
